# EUROPÄISCHE PATENTSCHRIFT

(11) **EP 1 257 523 B1**
(45) Veröffentlichungstag und Bekanntmachung des Hinweises auf die Patenterteilung: **21.06.2006**
(21) Anmeldenummer: 01911673.0
(22) Anmeldetag: 21.02.2001
(51) Int. Cl.: C07C 227/14

(54) **VERFAHREN ZUR HERSTELLUNG VON 3-AMINOALKANSÄUREESTERN**
METHOD OF PRODUCING 3-AMINOALKANOIC ACID ESTERS
PROCEDE DE PREPARATION D'ESTERS D'ACIDE 3-AMINOALCANOIQUE

(30) Priorität: 22.02.2000 EP 00103714; 12.05.2000 US 203902 P
(43) Veröffentlichungstag der Anmeldung: 20.11.2002
(73) Patentinhaber: Lonza AG, 4052 Basel (CH)
(72) Erfinder: FUCHS, Rudolf, CH-1950 Sion (CH)
(86) Internationale Anmeldenummer: PCT/EP2001/001955
(87) Internationale Veröffentlichungsnummer: WO 2001/062707

(56) Entgegenhaltungen:
- EP-A- 0 144 980
- BARTOLI, GIUSEPPE ET AL: "Chemo- and Diastereoselective Reduction of.beta.-Enamino Esters: A Convenient Synthesis of Both cis- and trans-.gamma.-Amino Alcohols and.beta.-Amino Esters" J. ORG. CHEM., Bd. 59, Nr. 18, 1994, Seiten 5328-5335, XP002171633
- DATABASE CHEMABS [Online] CHEMICAL ABSTRACTS SERVICE, COLUMBUS, OHIO, US; MURZAGULOVA, K. B. ET AL: "Commercial method for production of 2,5-dimethyl-4-piperidone" retrieved from STN Database accession no. 130:140761 XP002171634 & KHIM.-FARM. ZH. (1998), 32(9), 52-53 ,
- DATABASE WPI Section Ch, Week 199532 Derwent Publications Ltd., London, GB; Class E13, AN 1995-244725 XP002171635 & RU 2 026 289 C (ANZHERO-SUDZHENSK CHEM PHARM WKS), 10. Januar 1995 (1995-01-10) in der Anmeldung erwähnt

## Beschreibung

Die Erfindung betrifft ein Verfahren zur Herstellung von 3-Aminoalkansäureestern durch Hydrierung von 3-Amino-2-alkensäureestern.

Es sind mehrere Verfahren zur Hydrierung von 3-Amino-2-alkensäureestern bekannt RU-C-2 026 289 beschreibt die Hydrierung von 3-Aminocrotonsäureethylester in Gegenwart eines Nickel-Katalysators.
US-A-4 585 887 beschreibt die Herstellung von optisch aktiven 3-Aminoalkansäureestern, wobei ein β-Ketoester mit einem chiralen Amin umgesetzt wird und das daraus resultierende Enamin in Gegenwart eines Pt/C-Katalysators hydriert wird.

Die Hydriergeschwindigkeit und die Selektivität sind bei den oben genannten, bekannten Hydrierverfahren nicht zufriedenstellend.

Aufgabe der vorliegenden Erfindung war es, ein Verfahren zur Herstellung von 3-Aminoalkansäureestern aus 3-Amino-2-alkensäureestern bereitzustellen, wobei die Hydrierung rasch und mit hoher Selektivität verläuft.

Erfindungsgemäss wird diese Aufgabe durch das Verfahren nach Patentanspruch 1 gelöst.

Es wurde gefunden, dass die katalytische Hydrierung in Gegenwart einer starken Säure sehr schnell verläuft und 3-Aminoalkansäureester in hoher Ausbeute erhalten werden.

Das erfindungsgemässe Verfahren betrifft die Herstellung von 3-Aminoalkansäureestern der allgemeinen Formel 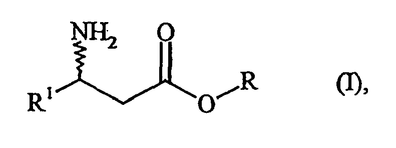 worin R C₁₋₆-Alkyl und R¹ Wasserstoff, C₁₋₆-Alkyl oder Phenyl bedeuten, oder deren Salzen,
durch katalytische Hydrierung der entsprechenden 3-Amino-2-alkensäureester der allgemeinen Formel 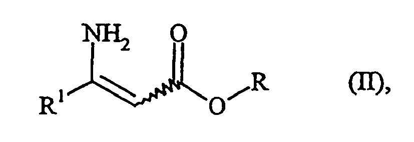 worin R und R¹ die oben genannten Bedeutungen haben.

Das Verfahren ist dadurch gekennzeichnet, dass die katalytische Hydrierung in Gegenwart einer starken Säure durchgeführt wird und das gebildete Salz des 3-Aminoalkansäureesters (I) und der starken Säure gegebenenfalls auf an sich bekannte Weise in den freien 3-Aminoalkansäureester (I) oder in ein anderes Salz übergeführt wird.

Unter C₁₋₆-Alkyl sind hier und im folgenden alle linearen oder verzweigten Alkylgruppen mit 1-6 Kohlenstoffatomen zu verstehen, wie beispielsweise Methyl, Ethyl, Propyl, Isopropyl, Butyl, Isobutyl, *sec*-Butyl, *tert-Butyl,* Pentyl, Isopentyl, *tert*-Pentyl, Neopentyl, Hexyl oder Isohexyl.

Der Rest R ist bevorzugt Methyl.

Unter starken Säuren sind hier und im folgenden Halogenwasserstoffsäuren wie beispielsweise Chlorwasserstoffsäure oder Bromwasserstoffsäure, Schwefelsäure, Sulfonsäuren wie beispielsweise Methansulfonsäure, Benzolsulfonsäure oder *p*-Toluolsulfonsäure und Perfluoralkansäuren zu verstehen.

Die starke Säure wird vorteilhaft in äquimolarer Menge oder im geringen Überschuss, bezogen auf den Ester, eingesetzt.

Eine bevorzugte Halogenwasserstoffsäure ist Chlorwasserstoffsäure.

Bevorzugte Sulfonsäuren sind Methansulfonsäure und p-Toluolsulfonsäure.

Eine bevorzugte Perfluoralkansäure ist Trifluoressigsäure.

Bevorzugte Katalysatoren sind Platin-, Palladium- oder Rhodium-Katalysatoren, insbesondere Trägerkatalysatoren. Besonders bevorzugt ist ein Platin-Trägerkatalysator.

Als Trägermaterialien sind alle gebräuchlichen Trägermaterialien geeignet, wie z. B. Aktivkohle, Aluminiumoxid, Siliciumdioxid, Silicium-Aluminiumoxid, Siliciumcarbid, Titandioxid, Magnesiumoxid oder Zeolithe. Bevorzugt ist Aktivkohle.

Die Trägerkatalysatoren enthalten vorteilhaft etwa 1-30 Gew%, vorzugsweise etwa 5-10 Gew% an Edelmetall.

Derartige Katalysatoren sind im Handel erhältlich, z. B. bei Degussa oder Heraeus.

Die Hydrierung wird vorzugsweise in einem wasserfreien Lösungsmittel durchgeführt.

Geeignet sind insbesondere Lösungsmittel aus der Gruppe der niedrigen Alkohole (z. B. Methanol. Ethanol), Ester (z. B. Essigsäuremethylester), Ether (z. B. Tetrahydrofuran, Dioxan, Diethylether) oder Halogenalkane (z. B. Dichlormethan, 1,2-Dichlorethan). Besonders bevorzugt ist wasserfreies Methanol.

Die Hydrierung erfolgt vorteilhaft bei Temperaturen von 0-150 °C und Drücken von 1-100 bar, vorzugsweise etwa bei Raumtemperatur (20-30 °C) und Drücken von 5-10 bar

Die 3-Amino-2-alkensäureester (II) können beispielsweise durch Umsetzung von Ammoniak mit 3-Oxoäkansäureestern, wie in der oben erwähnten russischen Patent-Veröffentlichung beschrieben, erhalten werden.

Eine weitere Möglichkeit zur Herstellung von 3-Amino-2-alkensäureestern (II) ist die in DE-A-2 425 705 beschriebene Ringöffnung von β-Lactamen und anschliessende Veresterung durch Umsetzung mit Alkohol/HCl.

Die Methansulfonate der 3-Ammonioalkansäureester der allgemeinen Formel 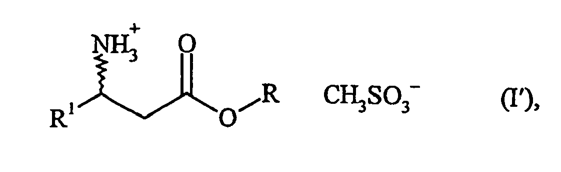 worin R und R¹ die oben genannten Bedeutungen haben, sind neu und ebenfalls Gegenstand der Erfindung.

Die Umwandlung des Salzes des 3-Aminoalkansäureesters (I) und der starken Säure in den freien 3-Aminoalkansäureester (I) kann auf an sich bekannte Weise durchgeführt werden, beispielsweise durch Zugabe einer Base wie Natronlauge.

Die folgenden Beispiele verdeutlichen die Durchführung des erfindungsgemässen Verfahrens, ohne dass darin eine Einschränkung zu sehen ist.

### Beispiel 1

### Herstellung von (RS)-3-Aminobuttersäuremethylester-Hydrochlorid (in Gegenwart von Chlorwasserstoffsäure)

In einem 160 ml Parr-Autoklaven wurden 5,0 g (43,4 mmol) 3-Aminocrotonsäuremethylester, 70 ml einer 9,3%igen Lösung von 17 g (43 mmol) HCl Gas in wasserfreiem Methanol und 0,22 g Pt/C Katalysator (Heraeus K0129) vorgelegt. Die Hydrierung erfolgte während 3 Stunden bei 21-25 °C unter 5-10 bar Wasserstoffdruck. Danach wurde der Katalysator abfiltriert und das Lösungsmittel am Rotationsverdampfer vollständig abdestilliert. Man erhielt 6,34 g reines (*RS*)-3-Aminobuttersäuremethylester-Hydrochlorid als Öl.

### Beispiel 2

### Herstellung von (RS)-3-Aminobutiersäuremethylester-Sulfat bzw. Hydrogensulfat (in Gegenwart von Schwefelsäure)

In einem 160 ml Parr-Autoklaven wurden 5,0 g (43,4 mmol) 3-Aminocrotonsäuremethylester, 70 ml wasserfreies Methanol, 1,5 Äquivalent Schwefelsäure und 0,22 g Pt/C Katalysator (Heraeus K0129) vorgelegt. Die Hydrierung erfolgte während 2 Stunden bei etwa 25 °C unter 3-10 bar Wasserstoffdruck. Danach wurde der Katalysator abfiltriert und das Lösungsmittel am Rotationsverdampfer vollständig abdestilliert. Man erhielt 6,13 g reines (*RS*)-3-Aminobuttersäuremethylester-Sulfat als Öl. Entsprechend kann das Hydrogensulfat hergestellt werden.

### Beispiel 3

### Herstellung von (RS)-3-Aminobuttersäuremethylester-Methansulfonat (in Gegenwart von Methansulfonsäure)

In einem 160 ml Parr-Autoklaven wurden 5,0 g (43,4 mmol) 3-Aminocrotonsäuremethylester, 70 ml wasserfreies Methanol, 1,0 Äquivalent Methansulfonsäure und 0,22 g Pt/C Katalysator (Heraeus K0129) vorgelegt. Die Hydrierung erfolgte während 2 Stunden bei ca. 20 °C unter 5-10 bar Wasserstoffdruck. Danach wurde der Katalysator abfiltriert und das Lösungsmittel am Rotationsverdampfer vollständig abdestilliert. Man erhielt 6,2 g reines (*RS*)-3-Aminobuttersäuremethylester-Methansulfonat als Öl.
Zur Charakterisierung wurde eine Probe aus Tetrahydrofuran kristallisiert.
Schmp.: 83-85 °C.

## Patentansprüche

1. Verfahren zur Herstellung von 3-Aminoalkansäureestern der allgemeinen Formel 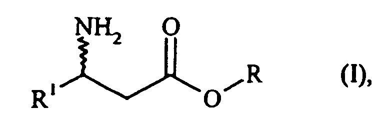 worin R C₁₋₆-Alkyl und R¹ Wasserstoff, C₁₋₆-Alkyl oder Phenyl bedeuten,
oder deren Salzen
durch katalytische Hydrierung der entsprechenden 3-Amino-2-alkensäureester der allgemeinen Formel 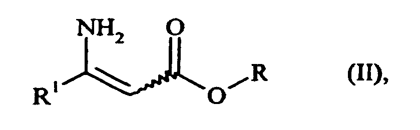 worin R und R¹ die oben genannten Bedeutungen haben, **dadurch gekennzeichnet, dass** die Hydrierung in Gegenwart einer starken Säure aus der Gruppe bestehend aus Halogenwasserstoffsäuren, Schwefelsäure, Sulfonsäuren und Perfluoralkansäuren durchgeführt und das gebildete Salz des 3-Aminoalkansäureesters (I) und der starken Säure gegebenenfalls auf an sich bekannte Weise in den freien 3-Aminoalkansäureester (I) oder in ein anderes Salz übergeführt wird.

2. Verfahren nach Anspruch 1, **dadurch gekennzeichnet, dass** als starke Säure Chlorwasserstoffsäure, Methansulfonsäure, *p*-Toluolsulfonsäure oder Trifluoressigsäure eingesetzt wird.

3. Verfahren nach Anspruch 1 oder 2, **dadurch gekennzeichnet, dass** als Katalysator ein Platin-, Palladium- oder Rhodium-Trägerkatalysator eingesetzt wird.

4. Verfahren nach Anspruch 3, **dadurch gekennzeichnet, dass** als Trägerkatalysator Platin auf Aktivkohle eingesetzt wird.

5. Verfahren nach einem der Ansprüche 1 bis 4, **dadurch gekennzeichnet, dass** R Methyl ist.

6. Verfahren nach einem der Ansprüche 1 bis 5, **dadurch gekennzeichnet, dass** die Hydrierung in einem wasserfreien Lösungsmittel durchgeführt wird.

7. Verfahren nach Anspruch 6, **dadurch gekennzeichnet, dass** als Lösungsmittel ein Lösungsmittel aus der Gruppe der niedrigen Alkohole, Ester, Ether oder Halogenalkane durchgeführt wird.

8. 3-Ammonioalkansäureester-Methansulfonate der allgemeinen Formel 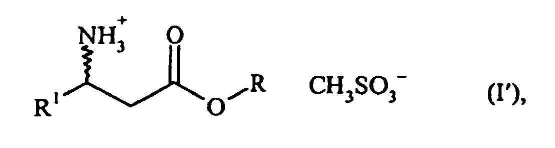 worin R und R¹ die in Anspruch 1 genannten Bedeutungen haben.

9. (*RS*)-3-Ammoniobuttersäuremethylester-methansulfonat.

## Claims

1. Process for the preparation of 3-aminoalkanoic acid esters of the general formula 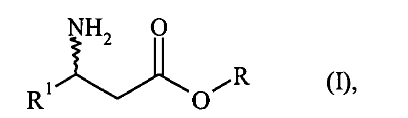 in which R is C₁₋₆-alkyl and R¹ is hydrogen, C₁₋₆-alkyl or phenyl, or their salts,
by catalytic hydrogenation of the corresponding 3-amino-2-alkenoic acid esters of the general formula 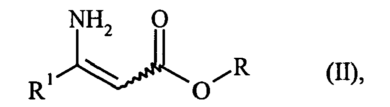 in which R and R¹ have the abovementioned meanings, **characterized in that** the hydrogenation is carried out in the presence of a strong acid from the group consisting of the hydrohalic acids, sulfuric acid, sulfonic acids and perfluoroalkanoic acids and the salt of the 3-aminoalkanoic acid ester (I) and the strong acid formed is optionally converted into the free 3-aminoalkanoic acid ester (I) or into another salt in a manner known *per se*.

2. Process according to Claim 1, **characterized in that** the strong acid employed is hydrochloric acid, methanesulfonic acid, p-toluenesulfonic acid or trifluoroacetic acid.

3. Process according to Claim 1 or 2, **characterized in that** the catalyst employed is a platinum, palladium or rhodium supported catalyst.

4. Process according to Claim 3, **characterized in that** the supported catalyst employed is platinum on activated carbon.

5. Process according to one of Claims 1 to 4, **characterized in that** R is methyl.

6. Process according to one of Claims 1 to 5, **characterized in that** the hydrogenation is carried out in an anhydrous solvent.

7. Process according to Claim 6, **characterized in that** the hydrogenation is carried out in a solvent from the group consisting of the lower alcohols, esters, ethers or haloalkanes.

8. 3-Ammonioalkanoic acid ester methanesulfonates of the general formula 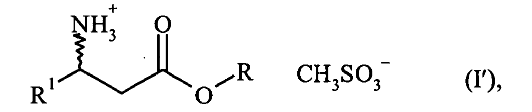 in which R and R¹ have the meanings mentioned in Claim 1.

9. Methyl (*RS*)-3-ammoniobutyrate methanesulfonate.

## Revendications

1. Procédé pour la préparation d'esters d'acides 3-aminoalcanoïques de formule générale 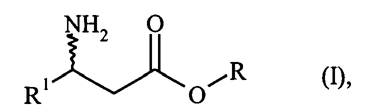 dans laquelle R représente un groupe alkyle en C₁-C₆ et R¹ représente un atome d'hydrogène, un groupe alkyle en C₁-C₆ ou phényle, ou de leurs sels,
par hydrogénation catalytique des esters d'acides 3-amino-2-alcénoïques correspondants, de formule générale 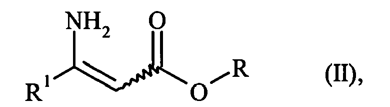 dans laquelle R et R¹ ont les significations données plus haut, **caractérisé en ce qu'**on effectue l'hydrogénation en présence d'un acide fort chois dans le groupe constitué par des hydracides halogénés, l'acide sulfurique, des acides sulfoniques et des acides perfluoroalcanoïques, et le sel formé de l'ester d'acide 3-aminoalcanoïque (I) et de l'acide fort est éventuellement converti, d'une façon connue en soi, en l'ester d'acide 3-aminoalcanoïque (I) libre ou en un autre sel.

2. Procédé selon la revendication 1, **caractérisé en ce qu'**on utilise comme acide fort l'acide chlorhydrique, l'acide méthanesulfonique, l'acide *p*-toluènesulfonique ou l'acide trifluoroacétique.

3. Procédé selon la revendication 1 ou 2, **caractérisé en ce qu'**on utilise en tant que catalyseur un catalyseur au platine, palladium ou rhodium fixé sur support.

4. Procédé selon la revendication 3, **caractérisé en ce qu'**on utilise comme catalyseur fixé sur support du platine sur charbon actif.

5. Procédé selon l'une quelconque des revendications 1 à 4, **caractérisé en ce que** R est le groupe méthyle.

6. Procédé selon l'une quelconque des revendications 1 à 5, **caractérisé en ce que** l'hydrogénation est effectuée dans un solvant anhydre.

7. Procédé selon la revendication 6, **caractérisé en ce qu'**on utilise comme solvant un solvant choisi dans le groupe des alcools inférieurs, des esters, des éthers ou des halogénoalcanes.

8. Méthanesulfonates d'esters d'acides 3-ammonioalcanoïques de formule générale 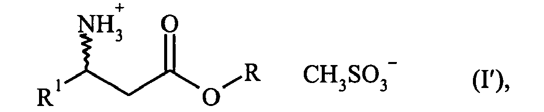 dans laquelle R et R¹ ont les significations données dans la revendication 1.

9. Méthanesulfonate de (*RS*)-3-ammoniobutyrate de méthyle.
